Europäisches Patentamt

**European Patent Office**

Office européen des brevets

(11) Numéro de publication: **0 155 198**
A1

(12)

# DEMANDE DE BREVET EUROPEEN

(21) Numéro de dépôt: 85400159.1

(51) Int. Cl.⁴: **C 12 N 15/00,** C 12 P 21/00

(22) Date de dépôt: **31.01.85**

(30) Priorité: **02.02.84 FR 8401614**

(71) Demandeur: **INSTITUT NATIONAL DE LA SANTE ET DE LA RECHERCHE MEDICALE (INSERM), 101, rue de Tolbiac, F-75654 Paris Cedex 13 (FR)**

(43) Date de publication de la demande: **18.09.85 Bulletin 85/38**

(72) Inventeur: **Yeramian, Patrick, 7, Avenue de Diane, F-94100 Saint Maur (FR)**
Inventeur: **Madaule, Pascal c/o Dr. Armand Tavitian INSERM, U 248, 10 Avenue Verdun, F-75010 Paris (FR)**
Inventeur: **Tavitian, Armand, 235, rue Lafayette, F-75010 Paris (FR)**

(84) Etats contractants désignés: **AT BE CH DE FR GB IT LI LU NL SE**

(74) Mandataire: **Warcoin, Jacques et al, Cabinet Régimbeau 26, avenue Kléber, F-75116 Paris (FR)**

(54) Vecteurs viraux de clonage et d'expression d'une protéine dans une cellule eucaryote, comportant au moins une partie du génome d'un rétro-virus; cellules eucaryotes transfectées; procédé utilisant de telles cellules et protéines obtenues.

(57) La présente invention concerne des vecteurs viraux de clonage et d'expression d'une séquence d'ADN codant pour une protéine déterminée dans une cellule eucaryote, caractérisés en ce qu'ils comportent au moins:
– une partie du génome d'un rétro-virus, ladite partie comprenant au moins la région promotrice et les éléments assurant la réplication, l'intégration sous forme de provirus, l'encapsidation du génome viral et l'épissage des RNA messagers viraux,
– et un site de restriction unique situé en aval de la région promotrice de sorte que la séquence d'ADN codant pour une protéine déterminée clonée à ce site soit placée sous le contrôle de cette région promotrice.
La présente invention concerne également les cellules eucaryotes transfectées par de tels vecteurs, un procédé de production de protéines utilisant de telles cellules et les protéines obtenues.

ACTORUM AG

VECTEURS VIRAUX DE CLONAGE ET D'EXPRESSION D'UNE PROTEINE DANS UNE CELLULE EUCARYOTE, COMPORTANT AU MOINS UNE PARTIE DU GENOME D'UN RETRO-VIRUS ; CELLULES EUCARYOTES TRANSFECTEES ; PROCEDE UTILISANT DE TELLES CELLULES ET PROTEINES OBTENUES.

La présente invention concerne de façon générale, des vecteurs viraux de clonage et d'expression d'une séquence d'ADN codant pour une protéine déterminée dans une cellule eucaryote, les cellules eucaryotes transfectées par de tels vecteurs, un procédé de production de protéines utilisant de telles cellules, ainsi que les protéines obtenues par la mise en oeuvre dudit procédé.

Plus particulièrement, la présente invention concerne la construction de vecteurs viraux de clonage et d'expression d'une séquence d'ADN codant pour une protéine déterminée dans une cellule eucaryote, lesdits vecteurs comportant au moins une partie du génome d'un rétro-virus et étant utiles, notamment, pour la confection de banques d'expression de DNA génomique et/ou de cDNA.

Dans la suite de l'exposé, dans un souci de commodité, on emploiera indifféremment les expressions "virus" ou "rétro-virus", "viral" ou "rétro-viral", pour désigner toute entité provenant d'un rétro-virus.

Jusqu'à présent, on a principalement utilisé dans la construction de vecteurs de clonage et d'expression, des réplicons autonomes dérivés de plasmides et/ou de bactériophages.

De tels réplicons se sont révélés des outils précieux et satisfaisants quant à l'étude du règne procaryote et aux "manipulations génétiques" réalisées sur les micro-organismes appartenant à ce règne : bactéries et levures essentiellement. Ils ont contribué dans une large mesure, à l'avancée récente de la biologie moléculaire et notamment, à l'établissement des techniques, devenues pour ainsi dire "classiques" aujourd'hui, d'hybridation du génome d'un organisme hôte par insertion dans ce dernier d'un fragment d'ADN d'un organisme donneur possédant une ou plusieurs propriétés intéressantes et recherchées.

Cependant, quels que soient les avantages de tels systèmes procaryotes, ils restent insuffisants dans l'étude des phénomènes plus complexes intervenant in vivo ou in vitro chez les eucaryotes - cellules de mammifères plus spécialement -, tels que l'épissage des gènes ou les phénomènes complexes de régulation.

Les vecteurs viraux sont dans cette optique un outil de premier choix. De nombreux virus étudiés extensivement ont prouvé leur capacité à être utilisés comme tels : SV 40, polyome, BPV, adénovirus, HSV, virus de la vaccine, rétrovirus, entre autre (réf. 36).

Chacun de ces virus selon sa biologie et son cycle réplicatif, impose certaines contraintes : spécificité d'espèce, taille du génome, génome cloné ou séquencé en totalité ou en partie, infection lytique, réplication épisomal intégration sous forme de provirus intégré (réf. 37).

Le choix du type de virus utilisé pour la construction de vecteur de gènes eucaryotes déterminés ne sera donc pas indifférent.

Les rétrovirus forment une famille comprenant tous les virus dont le cycle réplicatif implique une étape de rétrotranscription. Leur génome est constitué d'un ARN monocaténaire transcrit par une ADN-polymérase-ARN dépendante - reverse transcriptase - en un ADN proviral, ADN double brin d· 5000 à 9000 bases, qui s'intègre ensuite dans le génome de la cellule hôte infectée (réf. 45, 53).

Les principaux avantages des rétrovirus en tant que vecteurs de gènes eucaryotes, dérivent de leur cycle biologique particulier : promoteur puissant, intégration stable sous forme de provirus dans le génome hôte, infection non lytique, production de virus à haut titre, pas de barrière d'espèce.

C'est pourquoi la présente invention concerne des vecteurs viraux de clonage et d'expression d'une séquence d'ADN codant pour une protéine déterminée dans une cellule eucaryote caractérisés en ce qu'ils comportent au moins :

- une partie du génome d'un rétrovirus, ladite partie comprenant au moins la région promotrice et les éléments assurant la réplication, l'intégration sous forme de provirus, l'encapsidation du génome viral et l'épissage des RNA messagers viraux,

- et un site de restriction unique situé en aval de la région promotrice de sorte que la séquence d'ADN codant pour une protéine déterminée clonée à ce site soit placée sous le contrôle de cette région promotrice.

Plus précisément, un vecteur viral selon la présente invention comprend une partie du génome d'un rétrovirus, ladite partie comportant au moins successivement :

- une première séquence LTR,
- une séquence "leader" encadrée par des séquences consensus donneur et accepteur d'épissage,
- une deuxième séquence LTR,

ainsi que les séquences d'ADN viral adjacentes à ces séquences LTR et "leader".

En effet, le provirus qui est la forme obligatoire du virus pour que celui-ci puisse être intégré dans le génome de la cellule hôte infectée, est formé de deux séquences répétées LTR encadrant le génome viral. Ce dernier est constitué d'une succession de gènes codant pour diverses protéines nécessaires au cycle biologique du virus : protéines de structure du virion, glycoprotéines d'enveloppe, enzymes, par exemple.

Il est établit que le promoteur viral est contenu dans la séquence LTR5' et que la séquence "leader" située en aval de la LTR5' contrôle l'encapsidation du RNA génomique synthétisé à partir de la matrice constituée par le provirus.

On sait de plus, que les deux séquences LTR sont responsables de la stabilité de l'intégration du provirus dans le génome de l'hôte après transfection.

Ainsi, il apparaît que les régions du génome viral indispensables à la réplication, l'intégration sous forme provirale, l'encapsidation du RNA génomique et à la maturation des RNA messagers viraux, correspondent aux séquences LTR et à la séquence leader ainsi que leurs séquences adjacentes.

Selon un mode de réalisation préféré de l'invention, le rétrovirus utilisé pour la construction des vecteurs est un virus leucémogène, en particulier le virus murin de Moloney (Mo-MuLV). Le promoteur du Mo-MuLV est en effet l'un des plus puissants promoteurs eucaryotes connus.

Selon un autre mode de réalisation, un vecteur viral selon l'invention, comprend en outre, entre les deux séquences répétées LTR, le gène env - codant pour les glycoprotéines d'enveloppe du virus - et le site de restriction PstI du virus Mo-MuLV.

Un souci constant rencontré dans la construction de vecteurs de clonage est d'obtenir un ou plusieurs sites de restriction uniques de sorte qu'après digestion par une des enzymes de restriction correspondantes, on obtienne un seul fragment de restriction et qu'ainsi, après ligation avec le gène que l'on désire cloner, on récupère un seul type de vecteur recombinant ayant intégré au site voulu le gène déterminé.

C'est pourquoi un vecteur - objet de la présente invention, comporte au moins un site de restriction unique situé en aval de la région promotrice issue du génome viral afin de placer sous le contrôle de cette région promotrice, la séquence d'ADN codant pour la protéine déterminée que l'on désire cloner, exprimer et/ou produire.

De préférence, ce site de restriction unique est porté par un fragment d'ADN provenant du phage M13Mp8 (Biolabs).

Selon un mode de réalisation de l'invention, un vecteur comprend, en outre, tout ou partie de l'ADN d'un plasmide bactérien, ledit plasmide bactérien étant ligaturé à l'extérieur des deux séquences LTR encadrant l'ADN viral.

On utilise, en particulier, le plasmide pBR322 ou l'un de ses mutants. C'est ainsi que l'on prépare le vecteur viral, selon l'invention, pB6.

L'efficacité de ces vecteurs a été testée avec deux gènes marqueurs de sélection dominants : TK et Néo$^R$ et avec un gène d'intérêt biologique : gène HBS de l'hépatite B humaine.

Comme mutant du plasmide pBR322, on emploie de préférence un mutant ne comportant pas de site de restriction PstI dans le gène codant pour la résistance à l'ampicilline. Le choix d'un tel mutant permet de conserver simultanément le site de restriction PstI issu du génome viral comme site de restriction unique - on dote ainsi le vecteur d'un site de clonage supplémentaire - et le gène de résistance à l'ampicilline constituant une possibilité de "screening" des seuls vecteurs recombinants par culture sur milieu contenant de l'ampicilline. On prépare de cette façon le vecteur viral, selon l'invention, pURpB6.

Selon un autre mode de réalisation de la présente invention, un vecteur comprend, en outre, tout ou partie de l'ADN d'un phage, ledit fragment d'ADN phagique étant également ligaturé à l'extérieur des deux séquences LTR encadrant l'ADN viral comprenant ou non un insert plasmidique.

A cet effet, on utilise de préférence le phage λL47.1. On construit de cette façon le vecteur, selon l'invention, λp647.

De façon préférée, un vecteur selon la présente invention comporte, cloné à l'un de ses sites de restriction unique situé en aval de la région promotrice d'origine virale, au moins une séquence d'ADN codant pour une protéine déterminée.

En particulier, on effectue le clonage de la séquence d'ADN codant pour l'antigène de surface (HBS) de l'hépatite B humaine (HBH).

Un autre objet de la présente invention consiste en toute cellule eucaryote, notamment toute cellule eucaryote supérieure - une cellule de mammifère par exemple -, transfectée par vecteur viral conforme à l'invention, comportant ou un non une séquence d'ADN codant pour une protéine déterminée clonée en un de ses sites de restriction uniques.

Enfin, l'invention concerne un procédé de production d'une protéine, caractérisé en ce qu'on fait cultiver sur un milieu approprié une cellule eucaryote transfectée par un vecteur conforme à l'invention, et en ce qu'on isole ensuite la protéine produite.

De préférence, la protéine produite est l'antigène de surface de l'hépatite B humaine.

La composition des milieux de culture est connue de l'homme de métier. L'isolement de la protéine produite peut être effectuée par tout procédé après éclatement éventuel des cellules lorsque la protéine n'est pas excrétée.

Les exemples ci-après permettront de mettre en évidence d'autres caractéristiques et avantages de la présente invention.

Les figures ci-annexées permettront de mieux comprendre certains aspect de la présente invention :

- la figure 1 représente un schéma du génome du Mo-MuLV et un grossissement de la partie 5',

- la figure 2 représente la construction du vecteur viral pB6,

- la figure 3 illustre l'insertion des fragments de gènes codant pour les marqueurs TK et Néo[R] dans pB6 lors des tests d'efficacité de ce vecteur,

- la figure 4 illustre l'insertion des séquences codantes pour les gènes préS et S du virus de l'hépatite B humaine dans pB6 et B2M13,

- la figure 5 représente la construction du vecteur viral λp647,

- la figure 6 représente le vecteur viral pURpB6,

- et la figure 7 illustre la construction du vecteur pB7 dérivé du vecteur pB6.

EXEMPLE I - <u>Matériel et méthodes</u>

On rappelle un ensemble de techniques connues de l'homme de l'art. D'autres matériels et méthodes peuvent être envisagés, cependant, selon un mode de réalisation préféré de l'invention, on utilisera les techniques indiquées ci-après.

On les rassemble ici en vue d'alléger l'exposé des exemples suivants, relatifs plus spécifiquement, à la construction des vecteurs rétro-viraux proprement dits.

<u>Lignées cellulaires</u>

- NIH-3T3 fournie par S. Ruscetti (NIH Bethesda) fibroblastes de souris NIH réceptrice d'ADN dans les expériences de transformation du fait de deux propriétés importantes :

. efficacité de transfection élevée

. absence d'apparition de foyer de transformation spontané après culture prolongée à confluence. Ce critère n'est plus valable après 10 à 15 passages, les cellules doivent être alors sous-clonées.

- LTK⁻ (réf. 26) fournie par R. Axel (Columbia NY) fibroblastes de souris déficients pour l'activité thymidine kinase. Conservée en culture sous une pression de sélection de 50 µg/ml diamino 2-6 purine (Sigma).

- RD (réf. 1) isolée par Mac Allister à partir d'un rhabdomyosarcome d'une fillette. Cette lignée exprime l'oncogène N-Ras détecté par transformation de cellules NIH-3T3 (réf. 4 et 5).

- 78A1 (réf. 2) fournie et isolée par C. Bernard (St-Louis) fibroblastes de rat transformés par le complexe viral de Moloney.

- WDC et MAP1 deux lignées de mélanome humain isolées à partir de métastases ganglionaires par W.D. Carey (St-Louis).

- NRK cellules fibroblastiques de rein de rat. Ces cellules peuvent être infectées par les virus murins de type Moloney avec l'avantage de ne pas posséder de virus endogènes homologues.

- 3 lignées de carcinomes humains établies par S. A.AARONSON (NIH Bethesda) fournies par M. Barbacid (NCI Bethesda) (réf. 3) :

A 2233 carcinome du colon

A 2182 carcinome du poumon

A 1604 carcinome de la vessie

Ces lignées expriment l'oncogène Ki-Ras détecté par transformation de cellules NIH-3T3.

Toutes ces lignées sont cultivées à 37°C, 5 % $CO_2$ dans du milieu Eagle modifié selon Dulbecco supplémenté par 10 % SFV préalablement décomplémenté (30' à 56°C).

Souches bactériennes

- HB 101 hôte des plasmides utilisés souche d'E. coli RecA$^-$ SupE

- LA 101 hôte du phage λL47.1 et de ses recombinants, souche E. coli SupE SupF RecA$^+$

- DH1 souche de E. coli RecA$^-$ SupE utilisée dans la technique de transformation au rubidium chloride.

Plasmides

- pBR 322 (réf. 9)

- pMov3 (réf. 8) plasmide fourni par R. Jaenish contient au site EcoRI de pBR 322 le virus Mo-MuLV sous forme de provirus intégré.

-Blur 8 (réf. 10) fourni et construit par D. Schmid, l'insert cloné au site Hind III de pBR 322 correspond à une séquence répétitive humaine non détectée chez la souris dans les conditions habituelles d'hybridation.

- pcp 10 (réf. 11) fourni par P. Tiollais, contient le génome complet de l'HBV humain au site EcoRI de pBR 322.

- pUR 222 (réf. 12) dérivé de pBR 322 modifié par J. Feunten (IRSC, Villejuif) qui ne possède ni site PstI, ni séquences "poison" (réf. 13).

- pAGO (réf. 15) exprime le gène TK de HSV.

- IPB1 (réf. 16) fourni par R. Axel, exprime sous contrôle du promoteur du gène TK de HSV le gène bactérien du transposan Tn5 de <u>E. coli</u> conférant la résistance à la généticine (G418 sulfate Gibco).

<u>Bactériophage</u>

λL47.1 décrit par W.A. Loewen (réf. 30).

<u>Test virologique</u>

La production rétrovirale est étudiée par le dosage de l'activité reverse transcriptase du surnageant de culture (réf. 6).

<u>Purification d'ARN cellulaire eucaryote</u> (réf. 27 et28)

Les cellules sont lavées 3 fois au PBS 4°C, décollées au grattoir, centrifugées 2000 rpm 5' 4°C, au culot cellulaire on ajoute 5 volumes de : 5 M guanidium isothiocyanate, 5 mM sodium citrate, 0,1 M β-mercapto-éthanol, 50 mM tris pH = 8, 10 mM EDTA pH = 8, 0,5 % sarcosyl, puis 1 g de chlorure de césium pour chaque 2,5 ml de volume. Centrifuger 35000 rpm 10°C 12 h sur un coussin de 5,7 M chlorure de césium, 0,1 M EDTA pH = 8. Récupérer le culot de RNA dans 0,05 M acétate de sodium pH = 5,2, 1 mM EDTA, 0,5 % SDS, ajouter un volume égal de phénol/Chloroforme/ alcool isoamylique (25-24-1), chauffer 5' à 60°C et refroidir dans la glace, réextraire à nouveau et précipiter à l'alcool 70 % 0,1 M acétate de sodium pH = 5,2.

Sélection des ARN poly A[+] (réf. 29)

On utilise la technique de Kritosech par fixation spécifique des ARN poly A[+] sur une colonne d'oligo dT cellulose (Biosul) à forte concentration saline (NaCl 0,5 M). Deux passages successifs sont nécessaires.

Purification d'ADN cellulaire eucaryote (réf. 7)

Dans un falcon de 175 cm2, les cellules décollées par l'action de la trypsine sont lavées 2 fois par du PBS à 4°C, lysées par 8 ml de tampon : 10 mH tris pH = 8, 10 mM EDTA, 10 mM NaCl, 0,5 % SDS, 100 µg/ml protéinase K. Incubation 12 h à 37°C, extraction 2 fois au phénol/chloroforme, dyalise 5 jours contre NTE, précipitation alcool 70 % 0,1 M NaCl, redissolution dans l'eau bidistillée à la concentration de $1\gamma/\lambda$.

Purification d'ADN de plasmide

Production de masse et minipréparation ont été préparées selon Birboim et Dolly (réf. 31).

Electroélution d'ADN sur gel d'agarose

Le fragment du gel est placé dans un boudin de dialyse et électroélué sous fort voltage (supérieur à 100 V). Le DNA est alors purifié par passage sur une colonne échangeuse d'ion (DE52 cellulose, Whatman).

Culture de phage en milieu liquide et extraction de l'ADN

Absorption de $5.10^6$ phages et $5.10^8$ bactéries (1 DO à 600 nm correspond environ à $4.10^8$ bactéries) dans 5 ml de 10 mM tris pH = 7,6, 10 mM MgCl2, à 37°C pendant 30'. On ajoute 1 l de ML préchauffé à 37°C. On met à incuber 12 h à 37°C en agitant fortement. Le lendemain, on ajoute 10 ml de chloroforme pour compléter la lyse bactérienne et on centrifuge 2 fois 10' à 10000 rpm pour se débarrasser des débris, puis 12 h à 10000 rpm, 4°C pour culotter les phages. Le culot est repris dans 2 ml de 10 mM MgCl$_2$ et placé au sommet d'un gradient préformé discontinu de ClCs de densité successive : 1,3 ; 1,5 et 1,7 g/l ; on centrifuge à 4°C, 30000 rpm, 1 heure. La bande des particules phagiques est repérée au milieu de la zone de densité 1,5 et récupérée avec une aiguille au travers du tube. On centrifuge à nouveau en gradiant d'équilibre en ClCs de densité 1,5 g/l, 35000 rpm 24 h, 4°C. La bande de phages est récupérée comme précédemment. On dialyse contre 10 mM tris pH = 7,5, 10 mM MgCl$_2$, puis on ajoute EDTA qsp 10 mM final. On effectue 2 extractions au phénol/chloroforme, et on dialyse à nouveau contre NTE. Le DNA est finalement précipité à l'éthanol.

Préparation rapide du DNA de phage

On infecte $10^6$ phages avec $10^8$ bactéries comme décrit précédemment et on coule sur une boîte de Pétri dans de la gélose molle (0,7 % Bactoagar). On laisse incuber une nuit à 37°C, la lyse doit être totale. On ajoute 5 ml de 10 mM MgCl$_2$ et on laisse diffuser les particules à travers la gélose 12 h à 4°C. On prélève 400 µl ; on ajoute : 10 µl SDS 10 %, 50 µl tris 2 M pH=8,5, 50 µl EDTA 0,5 M pH = 8 ; on chauffe 5' à 70°C ; on ajoute 50 µl d'acétate de potassium 5 M , 30' dans la glace ; on centrifuge 15' et on précipite le surnageant à l'éthanol à 25°C ; on centrifuge le précipité, sèche et redissout le DNA dans le tampon approprié.

Transformation des bactéries HB 101 compétentes (réf. 32)

On utilise la méthode CaCl$_2$ 50 mM avec choc thermique 2' à 42°C (efficacité $10^3$ colonies par ng de plasmide super enroulé).

0155198

Transformation des bactéries DH1 : méthode au rubidium chlorure (réf. 33 et 34)

A partir d'une préculture fraiche de cellules DH1, on inocule 300 ml de milieu SOB : 2 % bactotryptone, 0,5 % extrait de levure, 10 mM NaCl, 10 mM $MgCl_2$, 10 mM $MgSO_4$, 2,5 mM KCl incubé à 37°C jusqu'à DO = 0,50. Ensuite les cellules sont refroidies dans la glace, peletisées à 5000 rpm 4°C 5' et resuspendues dans 1/3 du volume initial dans le tampon TFB : 10 mM K-MES, pH = 6,2, 100 mM RbCl, 45 mM $MnCl_2$, 10 mM $CaCl_2$, 3 mM $CoCl_2$, placées sur la glace à 4°C pendant 15', peletisées à nouveau et resuspendues dans 1/12,5 du volume initial. On ajoute : DMSO qsp 3,5 %, on incube 5' à 4°C : on ajoute DTU qsp 75 mM, on incube 10' à 4°C ; on ajoute une nouvelle dose de DMSO qsp 7 % final et on incube 5' à 4°C. A un aliquot de 210 µl, on ajoute le DNA ligaturé ( < 10 µl), on laisse incuber 30' à 4°C , on effectue un choc thermique 90' à 42°C et on refroidit dans la glace 2'. On ajoute 800 µl de SOC (milieu SOB additionné de 20 mM glucose) et on incube 1 h à 37°C. On dilue dans le volume nécessaire de SOB et on étale sur Agar boîtes de Pétri ML, Bactoagar 1,2 % contenant l'antibiotique choisi (ampicilline 0,1 mg/ml, tétracycline 0,075 mg/l).

Extraits d'encapsidation in vitro du

bactériophage $\lambda$ (réf. 35)

On utilise 2 souches bactériennes lysogènes pour un phage portant une mutation ambre dans différentes parties du génome :

BHB 2688 $RecA^-$ lysogène pour un phage $red^-$Eam Sam BHB 2690 $RecA^-$ lysogène pour un phage $red^-$Dam Sam Chaque souche est testée préalablement pour la lysogénie et le caractère $RecA^-$ (par la sensibilité aux UV).

- Lysat FTL (freeze thaw lysate) souche BHB 2688.

A partir d'une préculture de la veille, on inocule avec 500 ml de ML à une DO de 0,1 et on incube à 30°C jusqu'à ce que la DO atteigne 0,3. On réalise une induction à 45°C, et on continue l'incubation à 37°C pendant 2 h. On centrifuge 10000 rpm 10' 4°C et resuspend le culot dans 3 ml de : 50 mM tris pH = 8, 10 % sucrose, puis 150 l de : 2 mg/ml lysozyme, 0,25 M tris pH = 8. On immerge immédiatement dans l'azote liquide, puis on laisse fondre dans la glace. On ajoute 150 l de tampon d'encapsidation, on mélange, centrifuge 35000 rpm 1 h 4°C, répartit le surnageant en aliquot et congèle dans l'azote liquide.

- Lysat SE (sonicated extract) souche BHB 2690.

On procède au début de la même façon que pour le lysat FTE. Le culot cellulaire est dissout dans 3 ml de tampon : 20 mM tris pH = 8, 1 mM EDTA, 5 mM β-mercaptoéthanol. On effectue une sonication dans la glace par petits coups de 5" jusqu'à ce que la viscosité diminue sensiblement. On centrifuge 10000 rpm 10' 4°C, récupère 3 ml de surnageant, ajoute 3 ml de tampon d'encapsidation, et congèle en aliquot dans l'azote liquide. (tampon d'encapsidation : 6 mM tris pH = 8, 50 mM spermidine neutralisée avec 2 M tris pH = 8, 50 mM putrescine, 20 mm $MgCl_2$, 30 mM ATP neutralisé avec NaOH 1 M, 30 mM β-mercaptoéthanol).

- Encapsidation in vitro.

On fait fondre dans la glace un volume de lysat FTL pour 1,5 volume de lysatSE, mélange en ajoutant le DNA ligaturé concentré jusqu'à 0,2 γ/λ dans 10 mM tris pH = 8, incube 1 h, dissout dans 1 ml de : 10 mM tris pH = 7,5, 10 mM $MgCl_2$, ajoute une goutte de chloroforme, et on se débarrasse des débris par centrifugation 30'.

Transfection de cellules eucaryotes (réf. 19 et 20)

Technique de coprécipitation du DNA et de phosphate de calcium, adaptée de Graham et Van der Erb.

- Transfection de DNA chromosomique, induction de foyers de transformation sur NIH-3T3 SR.

Les cellules NIH-3T3 fraîchement décongelées sont passées 2 fois au 1/3, puis ensemencées à 700000 cellules par Pétri de 60 mm de diamètre (falcon). Dans un tube de Kahn stérile, on mélange : 500 µl de tampon de transfection 2X (0,28 M NaCl, 0,05 M Hepes Buffer, 1,5 mM $Na_2HPO_4$, le pH est ajusté à 7,04 exactement. Le pH du tampon de transfection est un élément fondamental de l'efficacité du tampon), DNA chromosomique stérilisé au chloroforme 40 µg, eau bidistillée qsp 1 ml final, $CaCl_2$ 2 M qsp 125 mM final. On laisse précipiter 20' à température ambiante, passe sur les cellules préalablement rincées avec du milieu frais et laisse 20' à température ambiante avant d'ajouter du milieu. Le lendemain matin, on effectue un choc au glycérol en incubant les cellules avec du milieu contenant 15 % de glycérol pendant 4'00. On rince abondamment. Le lendemain, 50 % des cellules sont mortes. On passe les cellules au 1/10ème. On renouvelle le milieu tous les 3 jours. Les foyers apparaissent après 5 à 7 jours avec le DNA des cellules 78A1 (qui sert de témoin de transfection) car il contient un virus sarcomatogène MSV capable de se répandre au voisinage de la cellule transfectée, il permet en outre de tester l'efficacité du tampon de transfection utilisé (de 4 à 6 foyers pour 20 γ de DNA). Avec le DNA de cellules exprimant un oncogène non viral, les foyers apparaissent après 2 à 3 semaines.

- Transfection de plasmides

La technique est identique, sauf que 4 h d'incubation des cellules avec le milieu de transfection sont suffisantes et que le choc au glycérol peut être omis. On utilise de 10 ng à 10 γ de plasmide superenroulé complété jusqu'à 20 γ avec du DNA "carrier" inerte (DNA chromosomique de E. coli HB 101, qui sert aussi de témoin négatif dans les expériences de transfection).

Sélection des cellules transfectées exprimant un marqueur dominant

Les drogues sont ajoutées    48 h après transfection :

- NéoR 400  γ/ml de généticine, puis 200  γ/ml dès que les colonies sont discernables (5 à 7 jours) (préparer 100 X dans l'eau).

- TK milieu HAT : hypoxanthine 15 μg/ml

aminoptérine 1  μg/ml

thymidine 5 μg/ml

(préparer 100 X dans 0,03 M NaOH).

Si l'on veut repiquer individuellement les colonies après transfection, il faut se placer dans des conditions telles qu'il n'apparaisse pas plus de 5 à 10 colonies par boîte de Pétri. Par exemple, on transfecte 10 ng de plasmide portant le gène TK, au 1,2 μg de plasmide portant le gène NéoR.

Isolement des foyers de transformations sur cellules NIH-3T3

Grâce à un cylindre de verre à l'intérieur duquel les cellules sont dissociées par une goutte de trypsine.

Isolement des colonies de cellules TK+ au NéoR

Grâce à une anse de platine enduite d'une goutte de trypsine.

Marquage au 32P par transfert de coupure (réf. 21)

On obtient une activité spécifique de 2 à 5.10^8 cpm/μg estimée sur une fraction aliquote TCA précipitable.

Transfert et hybridation des DNA selon Southern réf. (22)

Les échantillons de DNA coupé par l'enzyme de restriction choisie sont additionnés de sucroses et bleu de bromophénol et déposés sur un gel d'agarose 0,8 %. Ils sont ensuite soumis à une migration 40 mA pendant toute une nuit. Le gel est passé 5' dans l'eau, 15' dans HCl 0,5 M, 15' dans l'eau, 15' dans NaOH 0,5 M et neutralisé par 3 bains successifs dans 3 M NaCl 0,5 M tris pH = 8. On transfert sur une nitrocellulose, puis on rince et cuit le papier 80°C 2 h.

Pendant 12 h, on effectue une préhybridation à 42°C avec 250 µl/cm2 du tampon :

5SSC, 5 Denhart, formamide 50 %, 50 mM tampon, phosphate pH=6,5, 250 µg/ml DNA soniqué dénaturé.

L'hybridation est ensuite réalisée pendant 12 h à 42°C avec 100 µl/cm du tampon contenant : 4 volumes du tampon de préhybridation, 1 volume de 50 % sulfate dextran, sonde radioactive dénaturée par la chaleur à $10^6$ cpm/ml de tampon.

On rince : 4 fois 5' avec 2SSC, 0,1 % SDS ; 2 fois 15' à 50°C avec 0,1 SSC, 0,1 % SDS.

On sèche et autoradiographie sur film Kodak XO Mat entre 2 écrans renforçateurs à -70°C.

<u>Transfert et hybridation des ARN</u>

5 à 10 µg d'ARN poly $A^+$ sont dénaturés dans une solution de DMSO 50 %, glyoxal 1 M (désionisé extemporanément sur une résine échangeuse d'ions AG 501 x 8 (D) Biorad), 10 mM tampon phosphate pH = 7,2 : chauffés 30' à 50°C, puis soumis à électrophorèse à travers un gel d'agarose 1,25 % dans un tampon phosphate 10 mM pH = 7,2 à 50 mA pendant 5 h. La technique du transfert est identique à celle décrite précédemment, sauf que le gel n'est pas traité avant-transfert. La technique d'hybridation est identique.

<u>Hybridation in situ</u> (plage de lyse et
colonies bactériennes)

Sur une boîte de Pétri froide, on dépose un
filtre de nitrocellulose et laisse 5 à 10'. Puis on
dépose successivement, en laissant sécher sous la lampe
après chaque opération, sur une goutte des tampons
suivants :

| Plage de Lyse | Colonies bactériennes |
|---|---|
| 1,5 M NaCl, 0,1 M NaOH 1' | 0,5 M NaOH, 0,1 M NaCl 5' |
|  | 1,5 M NaCl               10' |
| 0,2 M tris pH = 7,5, 2 mM EDTA          1' | 0,2 M tris pH = 7,5, 2 mM EDTA          10' |
| 2SSC, 2 mM EDTA     2 x 1' | 2SSC, 2 mM EDTA     2 x 1' |

On sèche 30' à température ambiante, cuit
2 h à 80°C, préhybride dans 200 ml de 6SSC, 2 Denhart
12 h 68°C, hybride avec 1,5 ml par filtre de 2SSC,
1 Denhart, 0,5 % SDS 2mM EDTA, 25 mM $NaPHO_4$ pH = 7,2,
sonde radioactive 300 000 cpm, 12 h 68°C. On lave
4 fois 45' à 68°C dans 1 Denhart, 2SSC, 0,1 % SDS ;
puis à température ambiante 1 heure dans 50 %formamide,
2SSC, 0,5 % SDS ; enfin on effectue 3 lavages rapides
dans 2SSC.

EXEMPLE II - Virus de départ : virus leucémogène
murin de Moloney (Mo-MuLV)

Le virus Mo-MuLV est décrit sous sa forme provirale.

En effet, après pénétration du virus dans la cellule-hôte, l'ARN monocaténaire du virion est copié par la reverse-transcriptase associée au virion (réf. 46, 47) en un ADN double-brin constituant le provirus.

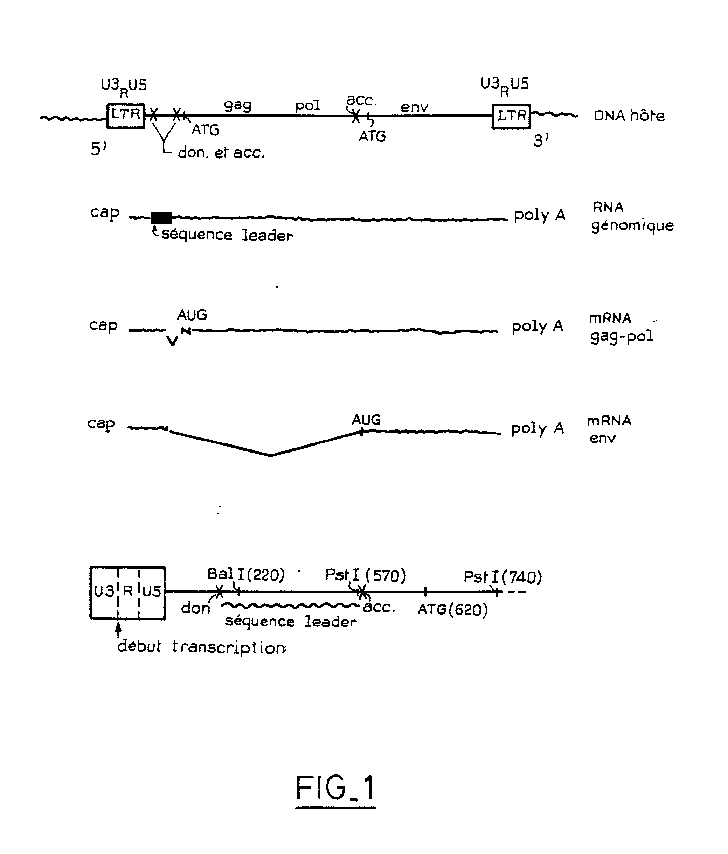

Le provirus (figure 1) est formé de deux séquences répétées LTR encadrant le génome viral. Celui-ci est constitué de trois gènes dénomés successivement gag, pol et env, codant respectivement pour les protéines de structure du virion, l'enzyme reverse-transcriptase et les glycoprotéines de l'enveloppe.

Une séquence LTR (Long Terminal Repeat ou séquence terminale répétée) se décompose elle-même en trois éléments juxtaposés U3, R et U5. Ces séquences comportent tous les signaux nécessaires à la transcription de l'acide ribonucléique et favorisent l'intégration du vecteur dans un ADN génomique de cellule-hôte.

Le provirus ainsi décrit est intégré dans le génome de la cellule-hôte et sert de matrice pour la synthèse des ARN génomique et sous-génomique (mRNA) sous le contrôle du promoteur viral contenu dans la séquence LTR de l'extrémité 5'.

Ces RNA sont "capés" et polyadénylés (réf. 55, 56).

Le RNA génomique est ensuite encapsidé sous le contrôle d'une courte séquence "leader" située en aval de LTR5' absente des RNA viraux.

Des séquences consensus donneur (don.) et accepteur (acc.) d'épissage permettent la maturation des RNA messagers viraux (fig. 1).

La séquence "leader" ainsi que les deux séquences répétées LTR constituent les éléments indispensables à la réplication, l'intégration sous forme de provirus et à l'encapsidation du génome viral.

En ce qui concerne le processus de synthèse du provirus, on se réfèrera au modèle proposé par Gilboa et Coffin (réf. 48, 49).

Un jour ou deux après l'infection des cellules-hôtes par le virus Mo-MuLV, l'état stationnaire est atteint, les cellules continuent à se diviser. 1 à 2 % de leur potentiel de synthèse est détourné au profit du virus. Le cycle réplicatif des rétrovirus n'est pas lytique, les virions sont continuellement produits par bourgeonnement de la membrane plasmique.

EXEMPLE III - Construction d'un vecteur rétro-viral de gène eucaryote dérivé du génome du virus leucémogène murin de Moloney (Mo-MuLV)

Le vecteur virus dont il est question ici est construit par délétion totale des séquences codantes pour les gènes gag et pol à partir de la forme provirale intégrée du virus de Mononey (Mo-MuLV) cloné par Jaenish au site EcoRI de pBR322. Ce vecteur conserve toutes les structures nécessaires à l'épissage des RNA viraux, à l'encapsidation, la réplication et l'intégration sous forme de provirus du RNA génomique viral.

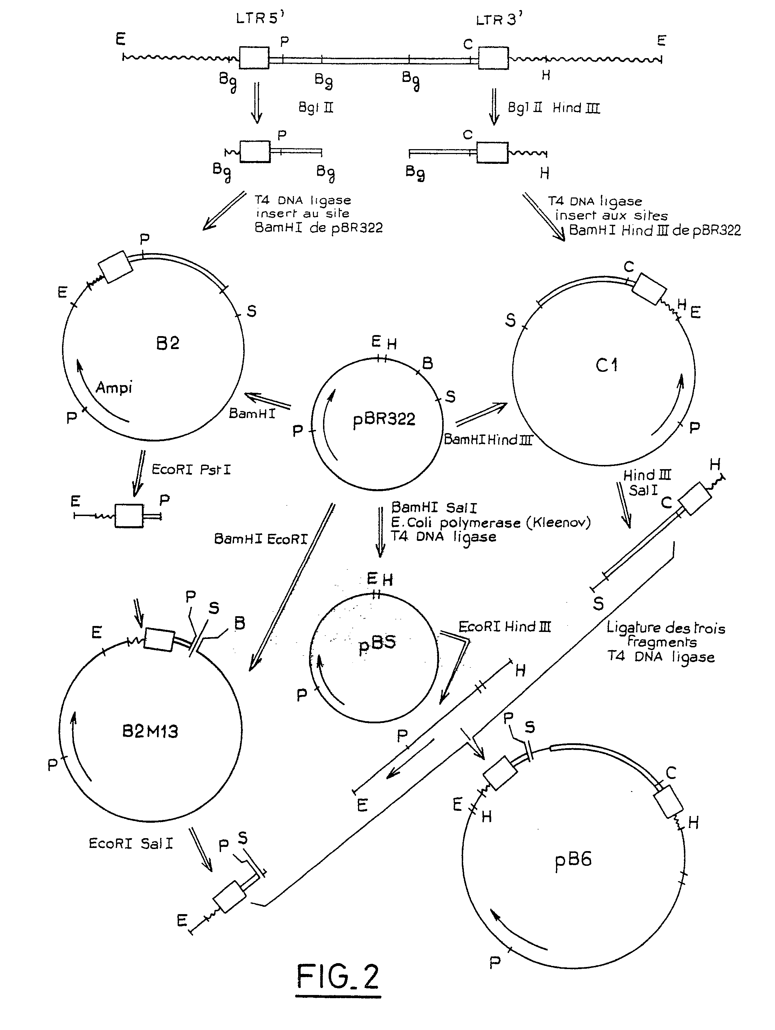

La construction de ce vecteur viral est illustré par la figure 2.

Le plasmide pMOV3 - contenant au site EcoRI de pBR322 le virus Mo-MuLV sous forme de provirus intégré tel que décrit à l'exemple II - est digéré par les enzymes de restriction Bgl II et HindIII.

On isole deux fragments : un fragment BglII BglII de 2,4 Kb et un fragment BglII HindIII de 4,0 Kb, les gènes gag et pol sont délétés.

Le fragment BglII BglII contient la séquence LTR5' et est sous-cloné au site BamHI de pBR322 pour former le clone B2. A partir du clone B2, on isole un fragment EcoRI PstI de 1,2 Kb. Celui-ci est sous-cloné aux sites EcoRI BamHI de pBR322 avec adjonction d'une courte séquence isolée à partir de la forme super-enroulée (super-coiled) du phage M13Mp8 (Biolabs) : on prépare ainsi le clone B2M13. Cette séquence est constituée de 16 nucléotides et contient les sites d'enzymes de restriction PstI, SalI et BamHI. Elle présente le double avantage de placer les sites BamHI et SalI qui serviront de site de clonage et de conserver dans la construction le site PstI du virus Mo-MuLV (position 570) qui est inclus dans une séquence accepteur d'épissage.

Le fragment BglII HindIII de 4,0 Kb contient la séquence LTR3' et le gène env. Ce fragment est sous-cloné aux sites BamHI HindIII de pBR322 pour former le clone CI.

Finalement, on isole à partir de B2M13 un fragment EcoRI SalI et à partir de CI un fragment HindIII SalI. Ces deux derniers fragments sont réunis aux sites EcoRI HindIII de pBR322 préalablement débarrassé de ses sites SalI et BamHI (plasmide pBS).

On prépare ainsi le plasmide pB6. Il a été adjoint 600 nucléotides en aval du promoteur viral (LTR), une courte séquence contenant les sites de restriction PstI et SalI. Des sites PstI et SalI, seul le site SalI est unique dans pB6 et servira de site de clonage ; les gènes clonés se trouvent donc en substitution des gènes viraux gag et pol.

La capacité maximale théorique du vecteur est de 6,7 Kb.

EXEMPLE IV - Expression des gènes marqueurs de
sélection TK et Néo[r] sous la dépendance du vecteur rétroviral

On teste l'efficacité du vecteur-viral pB6 avec
2 gènes marqueurs de sélection dominants : TK et Néo[r]. Le
gène TK (thymidine kinase) permet aux cellules TK[-] de se
multiplier en l'absence de thymidine et le gène Néo[r] (phosphotransférase) permet aux cellules de pousser en présence de
généticine (antibiotique G418 Gibco).

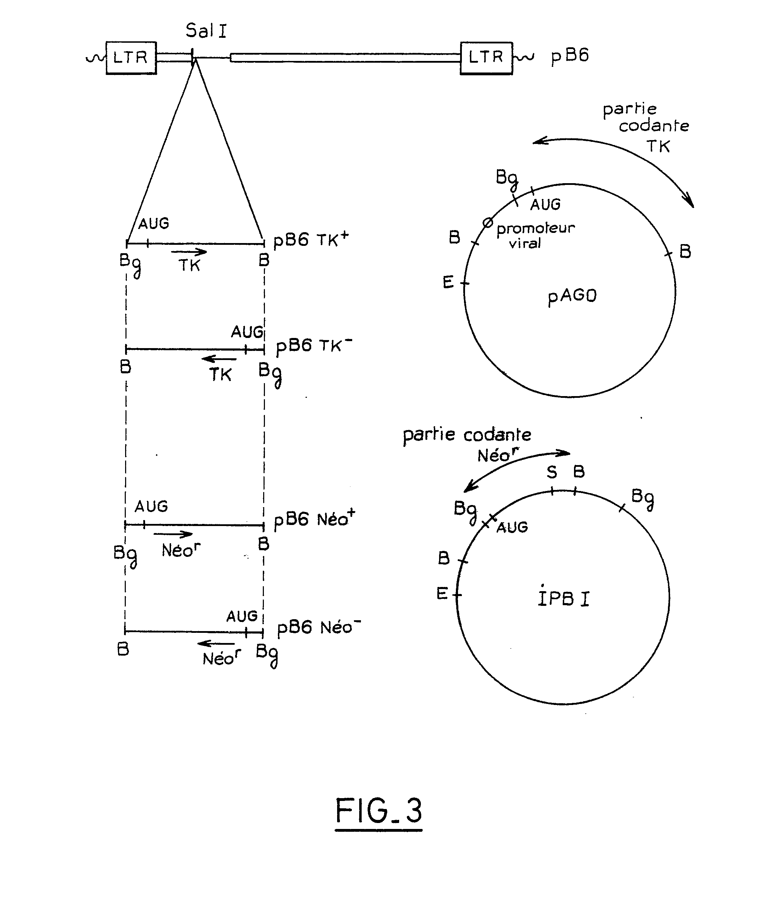

On place donc les séquences codantes des gènes
TK et Néo[r] sous la dépendance du promoteur viral (figure 3),
en substitution des gènes viraux gag et pol.

Pour ce faire, on utilise :

- le fragment BglII BamHI (2,3 Kb) du plasmide
pAGO qui contient la partie codante du gène TK de HSV sans
son promoteur propre,

- et le fragment BglII BamHI (1,2 Kb) du plasmide IPBI qui contient la partie codante du gène Néo[r] sans
le promoteur propre du gène TK.

Chacun de ces fragment est réparé par l'enzyme
de Kleenov et inséré au site SalI, préalablement également
traité par l'enzyme de Kleenov, du plasmide pB6.

L'orientation des gènes est déterminée par l'analyse du diagramme des enzymes de restriction.

Dans pB6 TK[+] et pB6 Néo[+], la direction de transcription naturelle de Mo-MuLV et des séquences codantes TK
ou Néo[r] est identique, dans pB6 TK[-] et pB6 Néo[-] la direction
de transcription est opposée. L'expression des gènes TK
sous la dépendance du vecteur viral est analysée par transfection de cellules LTK[-] (déficientes pour l'activité
thymidine kinase), suivie de sélection en milieu HAT des
cellules produisant un produit actif du gène TK.

L'expression du gène Néo$^r$ est analysée sur NIH-3T3 avec sélection par l'antibiotique G418.

Des colonies résistantes à la drogue apparaissent après transfection avec pB6 TK$^+$ ou pB6 Néo$^+$ (tableau 1).

Par contre, aucune colonie n'apparaît après transfection des cellules avec pB6 TK$^-$ ou pB6 Néo$^-$. Ceci suggère fortement que les gènes TK ou Néo sont bien exprimés sous l'influence du promoteur viral de Mo-MuLV.

## TABLEAU 1

| Plasmides Recombinants | Colonies HAT résistantes (cellules LTK$^-$) |
|---|---|
| pB6 TK$^+$ | 10 col/10 ng DNA/600.000 ç |
| pB6 TK$^-$ | 0 colonie |
| Plasmides Recombinants | Colonies résistantes à G418 (cellule NIH-3T3) |
| pB6 Néo$^+$ | 22 col/1,2 γ DNA/600.000 ç |
| pB6 Néo$^-$ | 0 colonie |

EXEMPLE V - Expression de l'antigène de
surface de l'hépatite B humaine

Le virus de l'hépatite B humaine (HBV) est un
virus à DNA double brin circulaire de 3100 pb, largement
répandu à travers le monde. Il pose un réel problème de
santé publique, surtour dans le Tiers-Monde avec haute
incidence d'hépatite chronique insidieuse et de cancer primaire du foie.

Les hépatocytes infectés synthétisent en excès
des enveloppes virales vides (ne contenant pas de DNA),
d'un diamètre de 22 nm. Cette particule présente dans le
plasma des porteurs chroniques est utilisée comme source
de vaccin. C'est un assemblage complexe glycoprotéolipidique dont le motif antigénique principal (HBS) est porté
par le produit du gène S. La région S du virus de l'hépatite B est divisée en deux parties : le gène S, proprement dit (nucléotides 155 à 883), précédé par une séquence
pré-S (nucléotides 2848 à 157) pouvant coder théoriquement pour 163 acides aminés.

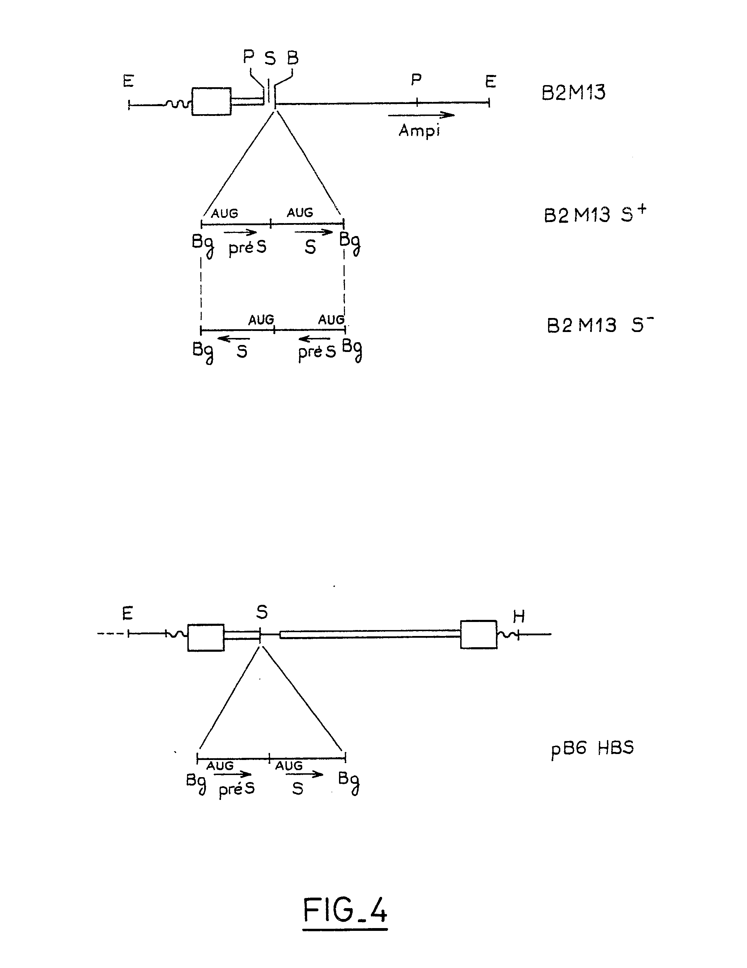

Deux types de construction sont effectuées (figure 4).

Dans un premier temps, on place sous le contrôle
du promoteur viral (LTR) de Mo-MuLV, le fragment BglII BglII
de HBV correspondant aux séquences codantes des gènes pré-S
et S. Ce fragment est cloné au site BamHI du clone B2M13.
Dans B2M13 $S^+$, la direction de la transcription naturelle
de Mo-MuLV et des séquences pré-S et S est identique.
Dans B2M13 $S^-$, la direction de transcription est opposée.

Dans un second temps, ce même fragment BglII BglII
de HBV est cloné au site SalI du vecteur viral pB6.

On étudie ensuite l'expression des gènes de
HBV clonés. Pour ce faire, des cellules $LTK^-$ sont cotrans- fectées par 10 ng de plasmide pAGO et 2 à 5 γ de vecteur viral recombinant : B2M13 S$^+$, B2M13 S$^-$ et pB6 HBS.

Les colonies apparues en 7 à 10 jours sont repiquées isolément à 21 jours et mises en culture de masse. La détection des particules HBS dans le surnageant de culture se fait par RIA (Abbott), les particules HBS étant visualisées en microscopie électronique sous forme de particules sphériques ou ovoïdes de 22 nm de diamètre. La production est appréciée quantitativement par dilution limite du surnageant de culture en supposant que la limite de détection en RIA est de 20 ng/ml.

Les résultats obtenus sont rassemblés dans le tableau 2. On a établi, ainsi des lignées cellulaires exprimant de façon stable d'importantes quantités d'antigène HBS.

TABLEAU 2

| Clone | Nombre de colonies TK$^+$ analysées | Colonies positives en RIA | Maximum de production obtenu |
|---|---|---|---|
| B2M13 S$^+$ | 11 | 8 | 640ng/24h/2 M cellules |
| B2M13 S$^-$ | > 20 | 0 | - |
| pB6 HBS | 20 | 2 | 300ng/24h/2 M cellules |

EXEMPLE VI - Construction d'un vecteur rétroviral apte à être utilisé dans la construction d'une banque d'expression génomique eucaryote

On clone dans le bactériophage λL47.1 le vecteur rétroviral décrit précédemment. Le bactériophage λL47.1 est un phage lambdoïde, qui possède une importante capacité d'insertion (24,1 Kb) avec une capacité minimale de 8,6 Kb ; le vecteur viral ne faisant que 6,4 Kb, on est obligé d'y adjoindre un insert de taille suffisante.

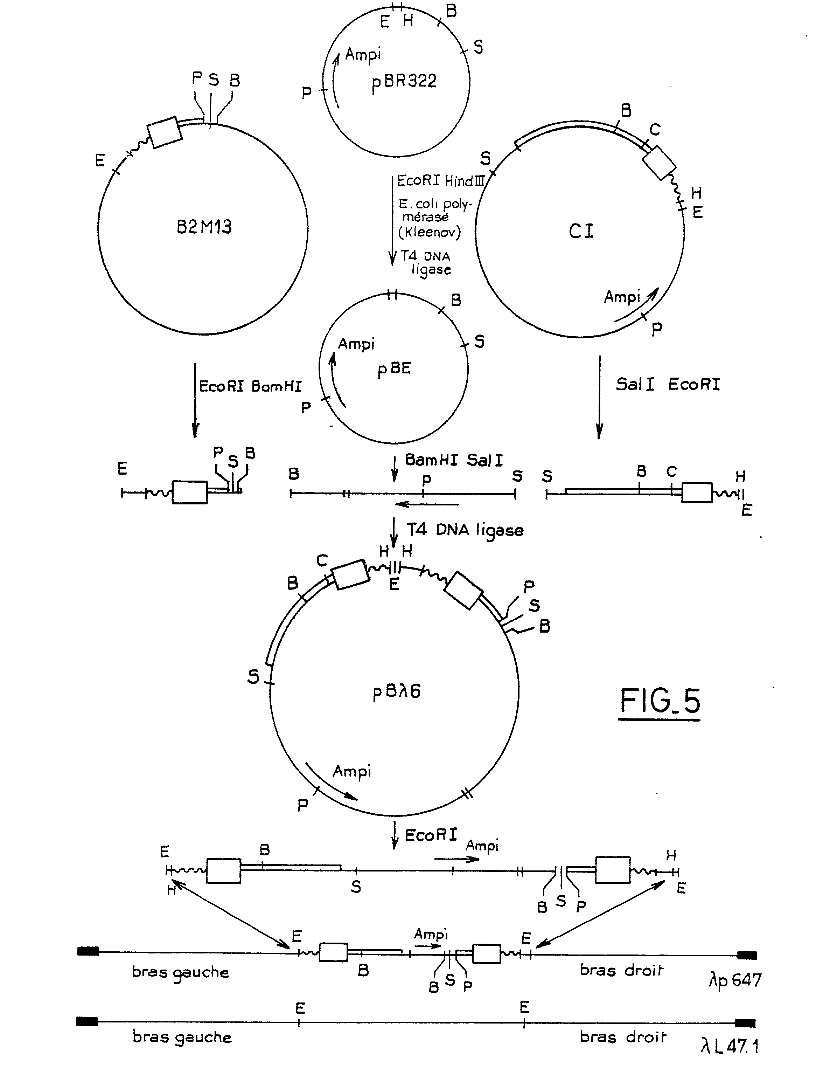

La construction du vecteur d'expression génomique λp647 est représentée à la figure 5.

pBR322 est digéré par les enzymes de restriction EcoRI et HindIII. L'action ensuite de E. coli polymérase (enzyme de Kleenov) et de T4 DNA ligase permet d'obtenir le plasmide pBE qui correspond ainsi à pBR322 débarrassé de ses sites EcoRI et HindIII.

On isole ensuite un fragment EcoRI BamHI de B2M13 et un fragment EcoRI SalI de CI tels que décrits à l'exemple III. Ces deux fragments sont réunis aux sites BamHI SalI de pBE. On obtient de cette façon le plasmide pBλ6.

Le plasmide pBλ6 est linéarisé par action de EcoRI (longueur 10,6 Kb) et est substitué dans le bactériophage λ47,1.

Pour cela, le phage λL47,1 est préalablement digéré par EcoRI. Les bras du phage sont isolés, et les fragments EcoRI EcoRI sont éliminés. Le plasmide pBλ6 également linéarisé et ligaturé avec les bras du phage λL47,1. Cette ligature est réalisée en présence d'un grand excès de bras du phage de façon à éviter au maximum l'intégration de deux plasmides pBλ6 en tandem.

Après encapsidation in vitro, l'analyse des phages recombinants λp647 est faite en trois étapes successives : hybridation in situ avec une sonde spécifique de gène env de Mo-MuLV, incapacité du phage recombinant de se propager sur des bactéries RecA⁻ (HB101), analyse de la carte de restriction.

On a ainsi construit un vecteur rétroviral apte à être utilisé pour la construction d'une banque d'expression génomique eucaryote. La banque génomique s'effectue au site BamHI de la construction, par digestion partielle du DNA génomique par les enzymes MboI ou Sau3A, en substitution de l'insert composé de PBE et d'une partie du gène env de Mo-MuLV. La capacité maximale théorique est de 17,1 Kb.

EXEMPLE VII - Construction d'un vecteur rétroviral apte à être utilisé pour
la construction d'une banque
d'expression de cDNA

La construction du vecteur rétroviral plasmidique est réalisée de la façon suivante.

Le plasmide pUR222 est préalablement digéré
par SalI.

Le plasmide pB6, tel que décrit à l'exemple III
est hydrolysé également par EcoRI, puis de façon très ménagée par PstI, ce qui permet d'isoler un fragment de 5,2 Kb.
Ce fragment, après avoir été soumis à l'action de la T4
polymérase, est sous-cloné au site SalI de pUR222.

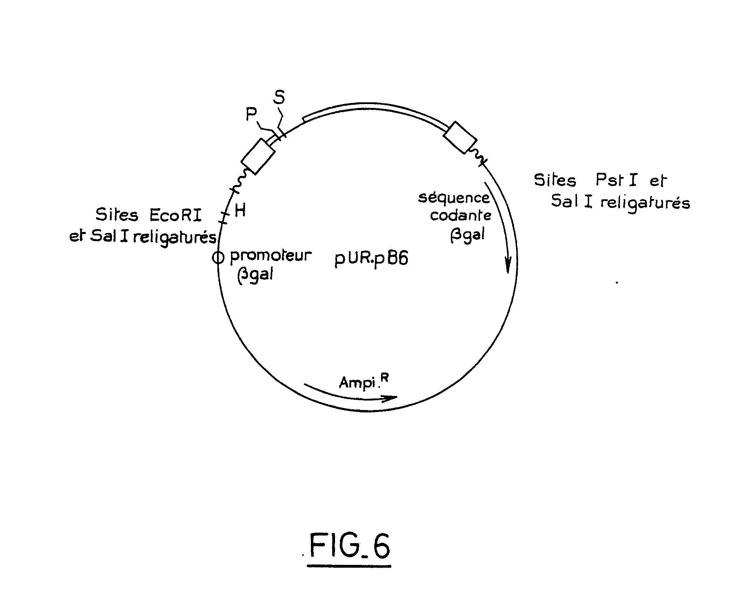

On prépare ainsi le plasmide pUR pB6 (figure 6),
dérivé du vecteur viral pB6. Il en diffère par deux caractères : la présence d'un site PstI unique en position 570,
qui peut donc servir de site de clonage du cDNA, l'absence
de "séquences poisons" de pBR322.

La construction d'une banque d'expression de
cDNA peut être réalisée de deux façons :

- au site PstI, la banque est construite par
"tailing" dC-dG,
- au site SalI, la banque est construite par
addition successive de "linker" SalI.

## EXEMPLE VIII

## CONSTRUCTION DU VECTEUR pB 7

Le vecteur pB 7 est construit à partir du vecteur pB 6, auquel on retire un fragment SalI-BamHI ; ce fragment comprend la partie 5' du gène env situé en amont du site BamHI et la courte séquence dérivée du plasmide pBR 322 insérée à l'intérieur du gène rétroviral au site SalI.

Le vecteur pB 6 débarrassé de ce fragment est ensuite religaturé avec une séquence "linker" SalI-BamHI pour donner le vecteur pB 7.

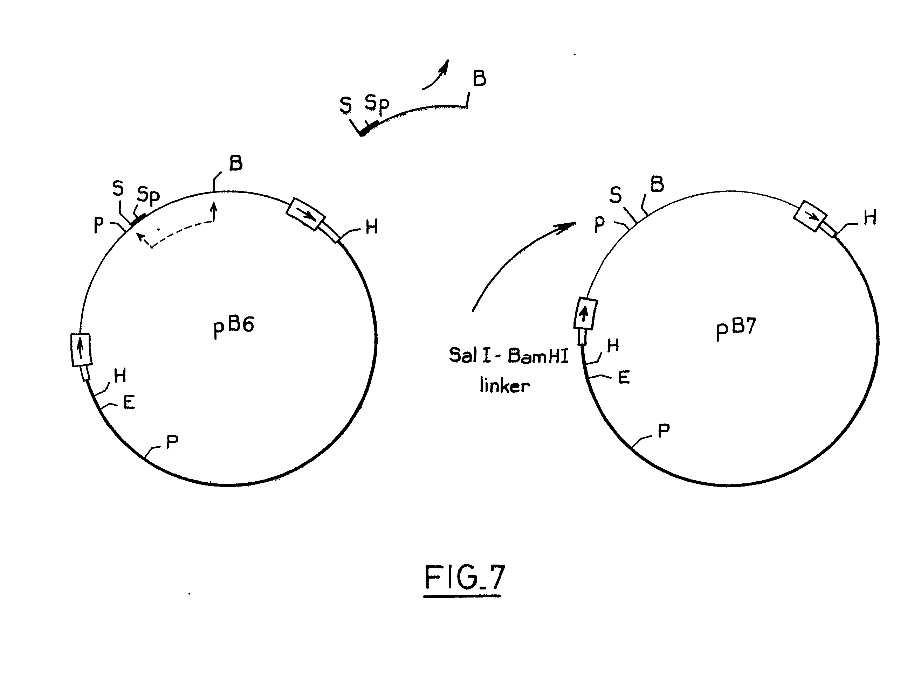

Sa construction est schématisée à la figure 7.

0155198

BIBLIOGRAPHIE

1. Mac Allister, R.M. et al. (1969) cancer 24 (3) 520-526.

2. Bernard, C., Boiron, M. et Lasneret, J. (1967) CRAS Paris D. 2170-2173

3. Barbacid, M. et al. (1982). Nature 300, 539-542.

4. Hall, A., Marshall, C.J., Spurr, N.K. et Weiss, R.A. (1982). Nature 303, 396.

5. Marshall, C.J., Hall, A. et Weiss, R.A. (1982). Nature 299, 171.

6. Tavitian, A., Hamelin, R., Olofsson, B. et Boiron, M. (1974). PNAS 71. 755-759.

7. Gross Bellard et al. (1973). Eur. J. Biochem. 36. 32-38.

8. Jähner, D. et Saenisch, R. (1980). Nature 287, 456.

9. Bolivar, F. et al. (1977). Gene 2. 95-113.

10. Rubin, C.H. (1980). Nature 888, 372-374.

11. Galibert, F. et al. (1979). Nature 281, 646-650.

12. Muller, Mill et al. (1981). Nuc. Acid Res. 9, 4087-4098.

13. Lusky, M., and Botcham, M. (1981) Nature 293, 79-81

14. Mulligan, R.C. et Berg. P. (1981). PNAS 78, 2072-2076.

15. Garapin et al. (1976). PNAS 76, 3757.

16. Rothstein et al. (1980). Cell 19, 795-805.

19. Graham et Van der Erb (1973). Virology 52, 456.

20. Graham et Van der Erb (1973). Virology 54, 536-539.

21. Rigby et al. (1977). J.M.B. 113, 237-251.

22. Southern, M.E. (1975). J.M.B. 98, 503-507.

26. Kits et al. (1963). Exp. Cell Biology 31, 291-312.

27. Glisin et al. (1974). Biochemistry 13, 2633.

28. Ulrich et al. (1977). Sciences 196, 1313.

29. Kritosech, A., Coutmon, M.L. et Kabat, D. (1975), J.B.C. 250, 6077.

30. Loenen, W.A. et al. (1980). Gene 10, 249-259.

31. Birboïm et Dolly (1979). Nuc. Acid Research 7, 1513.

32. Mandel, M. et Higa, A. (1970). J.M.B. 53, 154.

33. Kushner, S.R. (1978) genetic engineering (ed. H.B. Boyer and S. Nicosia) P. 17 Elsvier/North Holland Amsterdam

34. D. Hanahan (1983) Studies on transformation of E. coli with plasmids - JMB sous presse.

35. Hohn, B. et Murray, K. 1979). PNAS 74, 3259.

36. Rigby, P.N. (1983). J. Gen. Virol. 64, 255.

37. Molecular Biology of tumor virus. Cold Spring Harbor Laboratory Ed. (1982)

45. Varmus, H.E., Vogt, P.K., Bishop, J.M. (1978) PNAS 70, 3067-3071.

46. Baltimore, D. (1970). Nature 226, 1209

47. Baluda, M.A., Nayak D.P. (1970). PNAS 66, 329.

48. Gilboa, E. et al. (1979). Cell 18, 93-100

49. Coffin, J.M. (1979). J. Gen. Virol. 42, 1-26.

55. Lai, M.M.C., Duesberg, P.H. (1972). Nature 235, 383-386

56. Beemon, K.L., Keith, J.M. (1976). Animal Virology, p. 97. Academic Press, N.Y.

REVENDICATIONS

1. - Vecteur viral de clonage et d'expression d'une séquence d'ADN codant pour une protéine déterminée dans une cellule eucaryote, caractérisé en ce qu'il comporte au moins :

- une partie du génome d'un rétro-virus, ladite partie comprenant au moins la région promotrice et les éléments assurant la réplication, l'intégration sous forme de provirus, l'encapsidation du génome viral et l'épissage des RNA messagers viraux,

- et un site de restriction unique situé en aval de la région promotrice de sorte que la séquence d'ADN codant pour une protéine déterminée clonée à ce site soit placée sous le contrôle de cette région promotrice.

2. - Vecteur viral selon la revendication 1, caractérisé en ce que ladite partie du génome du rétro-virus comporte successivement au moins :

- une première séquence LTR,
- une séquence "leader" encadrée par des séquences consensus donneur et accepteur d'épissage,
- une deuxième séquence LTR, ainsi que les séquence d'ADN viral adjacentes à ces séquences LTR et "leader".

3. - Vecteur viral selon les revendications 1 ou 2, caractérisé en ce que le rétro-virus utilisé est un virus leucémogène, notamment le virus Mo-MuLV.

4. - Vecteur viral selon la revendication 3, caractérisé en ce qu'il comporte en outre entre les deux séquences répétées LTR, le gène env et le site de restriction PstI du virus Mo-MuLV.

5. - Vecteur viral selon l'une des revendications 1 à 4, caractérisé en ce que le site de restriction unique situé en aval de la région promotrice provient d'un fragment d'ADN du phage M13Mp8.

6. - Vecteur viral selon l'une des revendications 1 à 5, caractérisé en ce qu'il comporte, en outre, tout ou partie de l'ADN d'un plasmide bactérien ligaturé à l'extérieur des deux séquences LTR encadrant l'ADN viral.

7. - Vecteur viral selon la revendication 6, caractérisé en ce que le plasmide bactérien est pBR322, ou l'un de ses mutants.

8. - Vecteur viral selon la revendication 7, caractérisé en ce que le plasmide bactérien est un mutant de pBR322 ne comportant pas de site de restriction PstI dans le gène codant pour la résistance à l'ampicilline.

9. - Vecteur viral selon l'une des revendications 1 à 8, caractérisé en ce qu'il comporte, en outre, tout ou partie de l'ADN d'un phage ligaturé à l'extérieur des deux séquences LTR encadrant l'ADN viral comprenant ou non un insert plasmidique.

10. - Vecteur viral selon la revendication 9, caractérisé en ce que le phage est λL47.1.

11. - Vecteur viral selon l'une des revendications 1 à 10, caractérisé en ce qu'il s'agit de pB6.

12. - Vecteur viral selon l'une des revendications 1 à 10, caractérisé en ce qu'il s'agit de pURpB6.

13. - Vecteur viral selon l'une des revendications 1 à 10, caractérisé en ce qu'il s'agit de λp647.

14. - Vecteur viral selon l'une des revendications 1 à 10, caractérisé en ce qu'il s'agit de pB 7.

15. - Vecteur viral selon l'une des revendications 1 à 14, caractérisé en ce qu'il comporte cloné au site de restriction unique, au moins une séquence d'ADN codant pour une protéine déterminée.

16. - Vecteur viral selon la revendication 15, caractérisé en ce que ladite séquence d'ADN code pour l'antigène de surface de l'hépatite B humaine.

17. - Cellule eucaryote transfectée par un vecteur viral selon l'une des revendications 1 à 14.

18. - Cellule eucaryote transfectée par un vecteur viral selon les revendications 15 ou 16.

19. - Procédé de production d'une protéine caractérisé en ce qu'on fait cultiver sur un milieu approprié une cellule eucaryote transfectée selon la revendication 18, et en ce qu'on isole la protéine produite.

20. - Procédé selon la revendication 19, caractérisé en ce que la protéine produite est l'antigène de surface de l'hépatite B humaine.

1/7

FIG_1

FIG. 2

0155198

FIG_3

4/7

0155198

B2M13

B2 M13 S+

B2 M13 S⁻

pB6 HBS

FIG_4

FIG.5

Sites EcoRI
et Sal I religaturés

Sites Pst I et
Sal I religaturés

séquence
codante
βgal

promoteur
βgal

pUR.p B6

Ampi.R

FIG.6

FIG.7

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| Y | THE EMBO JOURNAL, vol. 1, no. 12, 1982, pages 1573-1578, IRL Press Ltd., Oxford, GB; C. STRATOWA et al.: "Recombinant retroviral DNA yielding high expression of hepatitis B surface antigen" * En entier * | 1,2,6, 7,14-19 | C 12 N   15/00 C 12 P   21/00 |
| Y | US-A-4 405 712  (G. VANDE WOUDE) * Revendications 1-10 * | 1,2,6, 7 | |
| A | CHEMICAL ABSTRACTS, vol. 99, no. 9, 29 août 1983, pages 174-175, no. 65319z, Columbus, Ohio, US; A.S. PERKINS et al.: "Design of a retrovirus-derived vector for expression and transduction of exogenous genes in mammalian cells" & MOL. CELL. BIOL. 1983, 3(6), 1123-1132 | 1 | |
| A | CHEMICAL ABSTRACTS, vol. 97, no. 9, 30 août 1982, page 153, no. 67178k, Columbus, Ohio, US; C.J. TABIN et al.: "The utilization of a retrovirus as a eukaryotic vector for transmitting cloned DNA sequences" & EUKARYOTIC VIRAL VECTORS, [CONF.], 1981, (Pub. 1982), 123-126 | | |

DOMAINES TECHNIQUES RECHERCHES (Int. Cl.4)

C 12 N

Le présent rapport de recherche a été établi pour toutes les revendications

| Lieu de la recherche | Date d'achèvement de la recherche | Examinateur |
|---|---|---|
| LA HAYE | 14-05-1985 | DELANGHE  L.L.M. |